(19) Europäisches Patentamt
European Patent Office
Office européen des brevets

(11) Publication number: **0 068 609**
**A1**

(12) **EUROPEAN PATENT APPLICATION**

(21) Application number: **82302359.3**

(22) Date of filing: **10.05.82**

(51) Int. Cl.³: **C 07 D 498/04**, A 61 K 31/42
//
(C07D498/04, 263/00, 205/00),
(A61K31/42, 31/43, 31/42,
31/545)

(30) Priority: **11.06.81 GB 8117995**

(43) Date of publication of application: **05.01.83**
**Bulletin 83/1**

(84) Designated Contracting States: **CH DE FR GB IT LI NL**

(71) Applicant: **BEECHAM GROUP PLC, Beecham House Great West Road, Brentford Middlesex (GB)**

(72) Inventor: **Stirling, Irene, 38 Harrison Close, Reigate Surrey (GB)**

(74) Representative: **Hesketh, Alan, Dr. et al, European Patent Attorney Beecham Pharmaceuticals Great Burgh Yew Tree Bottom Road, Epsom, Surrey, KT18 5XQ (GB)**

(54) **Derivatives of clavulanic acid, a process for their preparation and their use.**

(57) A class of clavulanic acid derivatives of formula (I):

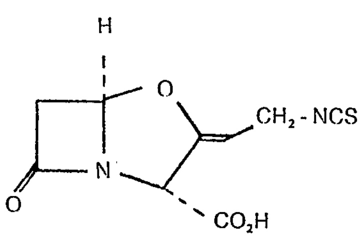

(I)

and salts and esters thereof, have antibacterial and β-lactamase inhibitory properties. The compounds are also valuable intermediates for preparing other clavulanic acid derivatives.



ACTORUM AG

0068609

# Derivatives of Clavulanic Acid, a Process for their preparation and their Use

This invention relates to derivatives of clavulanic acid and in particular to a class of derivatives substituted by an isothiocyanato group. These compounds have antibacterial and β-lactamase inhibitory properties, and therefore are of use in the treatment of bacterial infection either alone or in a synergistic composition with other antibacterial agents, such as penicillins and cephalosporins.

The present invention provides a compound of the formula (I):

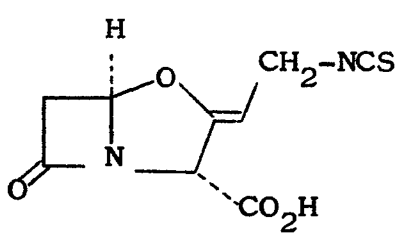

(I)

and salts and esters thereof.

Preferably the compounds of this invention are provided free from any other clavulanic acid derivative. Suitably the compound of the formula (I) or a salt or ester thereof is at least 50% pure, more suitably at least 75% pure and preferably at least 90% pure. The compounds of this invention are favourably in crystalline form.

The major utility of the compound of the formula (I), and salts and esters thereof, is as a pharmaceutical and accordingly the salts and esters of the compound of the formula (I) are preferably pharmaceutically acceptable. Both the pharmaceutically acceptable and non-pharmaceutically acceptable compounds of this invention may be used as chemical intermediates and also as antibacterial agents or β-lactamase inhibitors in non-pharmaceutical usage such as a disinfectant or paint additive. Suitable pharmaceutically acceptable salts of the compounds of the formula (I) include metal salts such as aluminium, alkali metal salts such as sodium and potassium, and alkaline earth metal salts such as calcium or magnesium; and ammonium and substituted ammonium salts, for example those with lower alkylamines such as triethylamine, trimethylamine and N-ethylpiperidine.

Suitable esters of the compounds of the formulae (I) include those cleavable by biological methods such as enzy matic hydrolysis, <u>in-vivo</u> hydrolysis, and those cleavable by chemical methods such as hydrogenolysis, hydrolysis, electrolysis or photolysis.

Suitably the carboxylic acid is esterified by a group of the sub-formula (a), (b), (c), (d), (e) or (f):

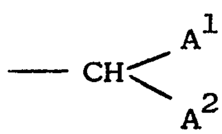 (a)

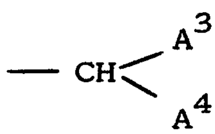 (b)

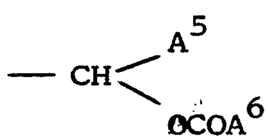 (c)

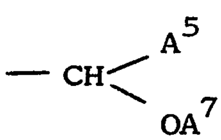 (d)

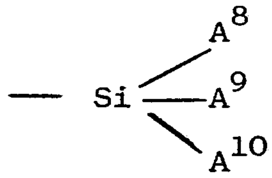

(e)

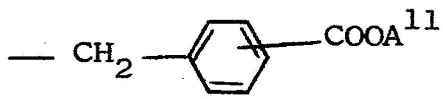

(f)

wherein $A^1$ is a hydrogen atom, $C_{1-6}$ alkanoyl or an $C_{1-5}$ alkyl group optionally substituted by $C_{1-7}$ alkoxy or $C_{1-7}$ carboxylic acyloxy, or an alkenyl or alkynyl group of up to 5 carbon atoms; $A^2$ is a hydrogen atom or a methyl group; $A^3$ is a phenyl group or a phenyl group substituted by a fluorine, chlorine or bromine atom or a nitro, $C_{1-3}$ alkyl or $C_{1-3}$ alkoxy group; $A^4$ is a hydrogen atom or a phenyl group or phenyl group substituted by a fluorine, chlorine or bromine atom or a nitro, $C_{1-3}$ alkyl or $C_{1-3}$ alkoxy group; $A^5$ is a hydrogen atom or a methyl group; $A^6$ is a $C_{1-4}$ alkyl, phenyl or $C_{1-4}$ alkoxy group or $A^5$ is joined to $A^6$ to form a phthalidyl, dimethylphthalidyl or dimethoxyphthalidyl group; $A^7$ is a $C_{1-4}$ alkyl, phenyl, chlorophenyl or nitrophenyl group; $A^8$ is a $C_{1-4}$ alkyl or phenyl group; $A^9$ is a $C_{1-4}$ alkyl or phenyl group; $A^{10}$ is $C_{1-4}$ alkyl; and $A^{11}$ is $C_{1-4}$ alkyl: or $CHA^1A^2$ is a phenacyl or bromophenacyl group.

Favourably $A^1$ is a hydrogen atom or a methyl, ethyl, vinyl or ethenyl group. Favourably $A^2$ is a hydrogen atom. Favourably $A^3$ is a phenyl, p-bromophenyl, p-methoxyphenyl or p-nitrophenyl group. Favourably $A^4$ is a hydrogen atom. Favourably $A^6$ is a methyl, t-butyl

or ethoxy group or is joined to $A^5$. Favourably $A^7$ is a methyl group.

Preferred groups of the sub-formula (a) include the methyl, ethyl and acetonyl groups.

Preferred groups of the sub-formula (b) include the benzyl and p-nitrobenzyl groups.

Preferred groups of the sub-formula (c) include the acetoxymethyl, pivaloyloxymethyl, α-ethoxycarbonyloxymethyl and phthalidyl groups.

A preferred group of the sub-formula (d) is the methoxymethyl group.

Preferred groups of the sub-formula (e) include the trimethylsilyl, tert-butyldimethylsilyl and tert-butyldiphenylsilyl groups.

A preferred group of the sub-formula (f) is p-methoxycarbonylbenzyl.

Particularly preferred esterifying groups are the p-nitrobenzyl and phthalidyl groups.

Pharmaceutically acceptable <u>in-vivo</u> hydrolysable esters are those esters which hydrolyse in the human body to produce the parent acid or its salt. Such esters may be identified by administration to a test animal such

as a rat or mouse by intravenous administration and thereafter examining the test animal's body fluids for the presence of the compound of the formula (I) or its salt.

Suitable esters of this type include those of sub-formula (c) as hereinbefore defined.

Further suitable esters include di($C_{1-6}$) alkylamino $C_{1-6}$ alkyl esters such as dimethylaminomethyl, dimethylaminoethyl, diethylaminomethyl and diethylaminoethyl.

Compounds of this invention when in crystalline form may be solvated, for example hydrated.

The present invention provides a pharmaceutical composition which comprises a compound of this invention and a pharmaceutically acceptable carrier.

The compositions of the invention include those in a form adapted for oral, topical or parenteral use and may be used for the treatment of the infection in mammals including humans.

Suitable forms of the compositions of this invention include tablets, capsules, creams, syrups, suspensions, solutions, reconstitutable powders and sterile forms suitable for injection or infusion. Such compositions may contain conventional pharmaceutically acceptable materials such as diluents, binders, colours, flavours, preservatives, disintegrant and the like in accordance with conventional pharmaceutical practice in the manner well understood by those skilled in the art of formulating anti-biotics.

Injectable or infusable compositions of a compound of the invention are particularly suitable as high blood levels of the compound can occur after administration by injection or infusion. Thus, one preferred composition aspect of this invention comprises a compound of the invention in sterile form and most suitably in sterile crystalline form.

The injectable solution of the compound of this invention may be made up in a sterile pyrogen-free liquid such as water, aqueous ethanol or the like.

An alternative approach to administering the compounds of this invention is to utilise an injectable suspension. Such suspensions may be made up in sterile water; sterile saline or the like, and may also contain suspending agents such as polyvinylpyrrolidone, lecithin or the like. Alternatively such compositions may be prepared in an acceptable oil suspending agent such as arachis oil or its equivalent. For use in such suspensions the compounds of this invention should be in the form of find particles.

Unit dose compositions comprising a compound of this invention adapted for oral administration form a further suitable composition aspect of this invention.

Unit dose compositions comprising a compound of this invention adapted for topical administration are also presented by this invention. In this instance 'topical administration' also includes local administration to internal surfaces of mammary glands of cattle, for example during the treatment of mastitis by intra-mammary administration.

The compound of the formula may be present in the composition as sole therapeutic agent or it may be present together with other therapeutic agents such as a penicillin or cephalosporin. Considerable advantages accrue from the inclusion of a penicillin or cephalosporin which shows instability to β-lactamases since the resulting composition shows enhanced effectiveness (synergy). Suitable penicillins, cephalosporins or other β-lactam anti-biotics for inclusion in such synergistic compositions include not only those

known to be highly susceptible to β-lactamases but also those which have a degree of intrinsic resistance to β-lactamases.

Suitable penicillins for inclusion in the compositions of this invention include benzylpenicillin, phenoxymethylpenicillin, carbenicillin, azidocillin, propicillin, ampicillin, amoxycillin, epicillin, ticarcillin, cyclacillin, pirbenicillin, azlocillin, mezlocillin, celbenicillin, pipericillin, and other known penicillins including pro-drugs therefor such as their *in vivo* hydrolysable esters such as the acetoxymethyl, pivaloyloxymethyl, α-ethoxycarbonyloxyethyl or phthalidyl esters of ampicillin, benzylpenicillin or amoxycillin, and aldehyde or ketone adducts of penicillins containing a 6-α-aminoacetamide side chain (such as hetacillin, metampicillin and analogous derivatives of amoxycillin) or α-esters of carbenicillin or ticarcillin such as their phenyl or indanyl α-esters.

Suitable cephalosporins for inclusion in the compositions of this invention include cefatrizine, cephaloridine, cephalothin, cefazolin, cephalexin, cephacetrile, cephamandole nafate, cephapirin, cephradine, 4-hydroxycephalexin, cefaparole, cephaloglycin, cefoperazone and other known cephalosporins or pro-drugs thereof.

Such compounds are frequently used in the form of a salt or hydrate of the like.

Naturally if the penicillin or caphalosporin present in the composition is not suitable for oral administration then the composition will be adapted for parenteral administration.

Highly favoured penicillins for use in the compositions of this invention include ampicillin,amoxycillin, carbenicillin and ticarcillin. Such penicillins may be used as a pharmaceutically acceptable salt such as the sodium salt. Alternatively the ampicillin or amoxycillin may be used in the form of find particles of the zwitterionic form (generally as ampicillin trihydrate or amoxycillin trihydrate) for use in an injectable suspension, for example in the manner hereinbefore described for a compound of this invention.

The preferred penicillin for use in the synergistic composition is amoxycillin, for example as its sodium salt or trihydrate.

Particularly suitable cephalosporins for use in the compositions of this invention include cephaloridine and cefazolin which may be in the form of a pharmaceutically acceptable salt for example the sodium salt.

When present together with a cephalosporin or penicillin, the ratio of a compound of the invention to the penicillin or cephalosporin agent may vary over a wide range of ratios, such as from 10:1 to 1:10 for example about 3:1, 2:1, 1:1, 1:2, 1:3, 1:4, 1:5 or 1:6, (wt/wt, based on pure free anti-biotic equivalent). Orally administrable compositions containing a compound of the invention will normally contain relatively more synergist than corresponding injectable compositions.

The total quantity of a compound of the invention in any unit dosage form will normally be between 25 and 1000 mg and will usually be between 50 and 500 mg, for example about 62.5, 100, 125, 150, 200 or 250 mg.

Compositions of this invention may be used for the treatment of infections of *inter alia* the respiratory tract, the urinary tract and soft tissues in humans and mastitis in cattle.

Normally between 50 and 3000 mg of the compounds of the invention will be administered each day of treatemnt but more usually between 100 and 1000 mg of the compounds of the invention will be administered per day, for example at 1-6 doses, more usually as 2, 3 or 4 doses. However, for the treatment of more severe systemic infections or infections of particularly intransigent organisms higher doses may be used in accordance with clinical practice.

The penicillin or cephalosporin in the synergistic composition of this invention will normally be present at approximately the amount at which it is conventionally used which will usually be expected to be from about 62.5 to 3000 mg per dose, more usually about 125, 250, 500 or 1000 mg per dose.

One particularly favoured composition of this invention will contain from 150 to 1000 mg of amoxycillin as the trihydrate or sodium salt and from 25 to 500 mg of a compound of this invention.

A further particularly favoured composition of this invention will contain from 150 to 1000 mg of ampicillin or a pro-drug therefor and from 25 to 500 mg of a compound of this invention.

Most suitably this form of composition will contain ampicillin trihydrate, ampicillin anhydrate, sodium ampicillin, hetacillin, pivampicillinhydrochloride,

bacampicillin hydrochloride, or talampicillin hydrochloride. Most suitably this form of the composition will contain a compound of the formula (I) when in crystalline form.

Most suitably the preceding composition will contain from 200 to 700 mg of the penicillin component. Most suitably the preceding composition will comprise from 50 to 250 mg of a compound of the formula (I) preferably in crystalline form.

Such compositions may be adapted for oral or parenteral use except when containing an in vivo hydrolysable ester of ampicillin or amoxycillin in which case the compositions will not be adapted for parenteral administration.

Another particularly favoured composition of this invention will contain from 200 to 2000 mg of carbenicillin, ticarcillin or a pro-drug therefor and from 50 to 500 mg of a compound of the invention.

Suitably this form of composition will contain di-sodium carbenicillin. Suitably this form of the composition will contain di-sodium ticarcillin.

More suitably this form of the composition will contain from 75 to 250 mg of a compound of the formula (I) preferably in crystalline form. Such compositions containing di-salts of carbenicillin and ticarcillin will be adapted for parenteral administration.

The present invention also provides a method of treating bacterial infections in humans or domestic mammals which comprises the administration of a compositions of this invention.

Commonly the infection treated will be due to a strain of Staphylococcus aureus, Klebsiella aerogenes, Escherichia coli, Proteus sp., Bacteroides fragilis or the like. The organisms believed to be most readily treated by an antibacterially effective amount of a compound of this invention is Staphylococcus aureus. The other organisms named are more readily treated by using a synergistically effective amount of the compound of the invention and a penicillin or cephalosporin. The administration of the two components may take place separately but in general we prefer to use a composition containing both the synergist and the penicillin or cephalosporin.

The indications for treatment include respiratory tract and urinary tract infections in humans and mastitis in cattle.

The present invention also provides a process for the preparation of a compound of the formula (I) or a salt or ester thereof, which process comprises the reaction of a compound of the formula (III):

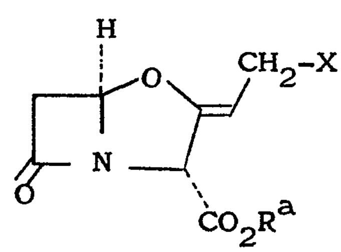

(III)

wherein $R^a$ is a carboxy-protecting group, and X is a displaceable group, with a source of thiocyanate ion; and thereafter if necessary:

i) removing any carboxy-protecting group $R^a$,

ii) converting the product into a salt or ester.

Suitably the source of thiocyanate ion is a metal thiocyanate, for example an alkali metal thiocyanate such as potassium thiocyanate or sodium thiocyanate. In an alternative the source of thiocyanate may be a polymer-supported thiocyanate such as a macroporous resin.

Suitably X is a readily displaceable group, for example halo such as chloro or bromo, or a sulphonyloxy moiety, for example $C_{1-6}$ alkyl or aryl-sulphonyloxy, such as a mesylate or tosylate, or X is a carboxylate moiety, for example a $C_{1-6}$ alkanoyloxy group optionally substituted by 1 to 3 halogen atoms, in particular acetoxy, dichloroacetoxy and trichloroacetoxy.

Suitably the reaction of the compound of the formula (III) with the source of thiocyanate will occur in an aprotic solvent, such as dimethylformamide or acetonitrile, at a non-extreme temperature for example between $-50^{o}C$ and $+50^{o}C$, more usually between $-10^{o}C$ and $+30^{o}C$ and preferably at ambient temperature.

Suitably carboxy-protecting groups $R^a$ include ester derivatives of the carboxylic acid. The derivative is

preferably one which may be readily cleaved at a later stage of the reaction.

Suitable ester-forming carboxy-blocking groups are those which may be removed under conventional conditions, and include those of sub-formulae (a) - (f) as hereinbefore defined. Suitable groups $R^a$ include benzyl, methyl, ethyl, p-methoxybenzyl, 2,4,6-trimethylbenzyl, 3,5-di-t-butyl-4-hydroxybenzyl, benzoylmethyl, p-nitrobenzyl, 4-pyridylmethyl, allyl, 2-methylallyl, 2,2,2-trichloroethyl, 2,2,2-tribromoethyl, acetonyl, t-butyl, diphenylmethyl, triphenylmethyl, 2-benzyloxyphenyl, 4-methylthiophenyl, methoxymethyl, t-amyl, tetrahydropyran-2-yl, tetrahydrofur-2-yl, a silyl, stannyl or phosphorus - containing group or an <u>in-vivo</u> hydrolysable ester.

The carboxy group may be regenerated from any of the above esters by usual methods appropriate to the particular $R^a$ group, for example base-catalysed hydrolysis, enzymically-catalysed hydrolysis, photolysis or hydrogenation.

The following Examples illustrate the invention.

Example 1

p-Nitrobenzyl 9-isothiocyanatodeoxyclavulanate

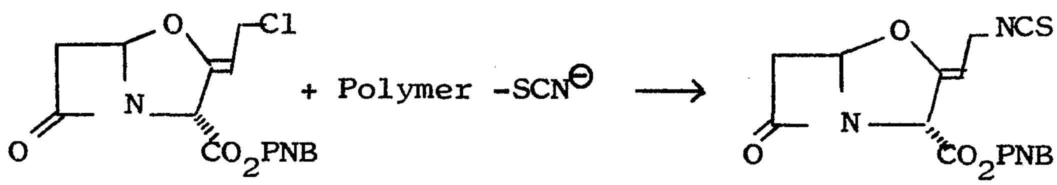


A solution of p-nitrobenzyl 9-chlorodeoxyclavulanate (1.5 g; 4.25 mmol) in dimethylformamide (50 ml) was treated with polymer-supported thiocyanate (5 g; activity ~ 9 mmol), previously dried azeotropically with toluene, (prepared by treating the chloride form of Amberlyst A26, a macroporous resin with quaternary ammonium groups, with aqueous potassium thiocyanate). The suspension was stirred at room temperature for 5 hours; the polymer was filtered off and washed with dimethylformamide. The filtrate and washings were combined and evaporated in vacuo. The title compound was purified by column chromatography on silica gel with cyclohexane: ethyl acetate, 2:1, as eluent. The product was obtained as a colourless crystalline solid ($CCl_4$) in 55% yield. Rf ($SiO_2$/cyclohexane: ethyl acetate, 1:1)=

0.42.

$\nu_{max}$ (Nujol) 2100 - 2180 (multiplet), 1780, 1760, 1690 $cm^{-1}$. δ ($CDCl_3$) 3.12 (1H, d, J 17 Hz, 6β-C$\underline{H}$), 3.54 (1H, dd, J 3 and 17 Hz, 6 α-C$\underline{H}$), 4.16 (2H, d, J 7 Hz, 9-C$\underline{H}_2$), 4.82 (1H, bt, J 7 Hz, 8-C$\underline{H}$), 5.15 (1H, bs, 3-C$\underline{H}$), 5.29 (2H, s, C$\underline{H}_2$Ar), 5.73 (1H, bd, J 3 Hz, 5-C$\underline{H}$), 7.52, 8.27 (4H, ABq, Ar-$\underline{H}$).

The p-nitrobenzyl ester group is removed by treatment with zinc in aqueous hydrochloric acid to give 9-isothiocyanatodeoxyclavulanic acid.

CLAIMS :

1. A compound of formula (I) or a salt or ester thereof:

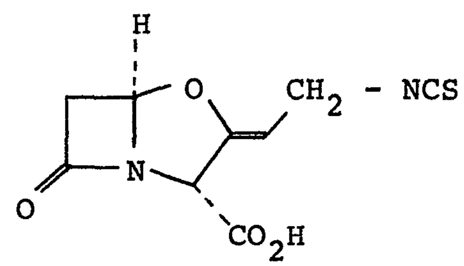

(I)

2. A compound as claimed in claim 1 in the form of a salt.

3. A compound as claimed in claim 2 in the form of an alkali metal, alkaline earth metal, ammonium or substituted ammonium salt.

4. A compound as claimed in claim 1 in the form of the free acid or a pharmaceutically acceptable salt or pharmaceutically acceptable ester thereof.

5. A compound as claimed in claim 1 in the form of an ester cleavable by chemical means.

6. A compound as claimed in claim 5 wherein the ester radical is a benzyl group optionally substituted with fluorine, chlorine, bromine, nitro, $C_{1-3}$ alkyl, or $C_{1-3}$ alkoxy.

7. A compound as claimed in claim 6 in the form of the p-nitrobenzyl ester.

8. A pharmaceutical composition which comprises a compound as claimed in claim 2 and a pharmaceutically acceptable carrier.

9. A composition as claimed in claim 8 which further comprises a penicillin or cephalosporin.

10. A process for the preparation of a compound as claimed in claim 1 which process comprises reacting a compound of formula (III):

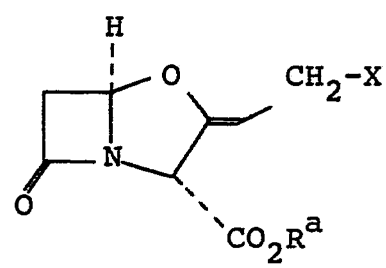

(III)

wherein $R^a$ is a carboxy-protecting group, and X is a displaceable group, with a source of thiocyanate ion; and optionally thereafter:

i) removing any carboxy-protecting group $R^a$; and

ii) converting the product into a salt or ester.

European Patent Office

0068609
Application number
EP 82 30 2359

# EUROPEAN SEARCH REPORT

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 3) |
| A | GB-A-1 579 531 (GLAXO) *Claims 1,22,36,45,51,52,115-118,138* | 1,8-10 | C 07 D 498/04<br>A 61 K 31/42 //<br>(C 07 D 498/04<br>C 07 D 263/00<br>C 07 D 205/00 )<br>(A 61 K 31/42<br>A 61 K 31/43<br>A 61 K 31/42<br>A 61 K 31/545) |
| | --- | | |
| A | DE-A-2 747 599 (GLAXO) *Claims 1-9,24-27* | 1,8-10 | |
| | --- | | |
| A | GB-A-1 585 124 (GLAXO) *Claims 1,24,44* | 1,8-10 | |
| | ----- | | TECHNICAL FIELDS SEARCHED (Int. Cl. 3) |
| | | | C 07 D 498/00<br>A 61 K 31/00 |

The present search report has been drawn up for all claims

| Place of search | Date of completion of the search | Examiner |
|---|---|---|
| THE HAGUE | 14-09-1982 | CHOULY J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82